# EUROPEAN PATENT SPECIFICATION

(11) **EP 0 670 844 B1**
(45) Date of publication and mention of the grant of the patent: **01.10.1997**
(21) Application number: 94900135.8
(22) Date of filing: 11.11.1993
(51) Int. Cl.: C07H 15/04, C07C 235/08, C07C 235/10, C11D 1/52

(54) **ALDONAMIDES AND THEIR USE AS SURFACTANTS**
ALDONAMIDE UND IHRE VERWENDUNG ALS GRENZFLÄCHENAKTIVE SUBSTANZEN
ALDONAMIDES ET LEUR UTILISATION COMME TENSIOACTIFS

(30) Priority: 25.11.1992 US 981644
(43) Date of publication of application: 13.09.1995
(73) Proprietor: UNILEVER PLC, London EC4P 4BQ (GB); UNILEVER N.V., 3013 AL Rotterdam (NL)
(72) Inventor: VERMEER, Robert, Nutley, NJ 07110 (US); AU, Van, Peekskill, NY 10566 (US); HARIRCHAIN, Bijan, South Orange, NJ 07079 (US)
(74) Representative: Fransella, Mary Evelyn
(86) International application number: EP9303171
(87) International publication number: WO9412511

(56) References cited:
- EP-A- 0 312 087
- EP-A- 0 550 106
- EP-A- 0 550 281
- EP-A- 0 551 675
- WO-A-89/10121
- WO-A-92/06155
- WO-A-92/06160
- CHEMISTRY LETTERS no. 4 , April 1990 , TOKYO JP pages 675 - 678 N. HIGASHI ET AL 'Permeation characteristics of a glycolipid monolayer-deposited agar hydrogel.'

## Description

The present invention relates to the use as surfactants and foaming agents of specific aldonamide compounds which are higher foaming and more soluble relative to other aldonamide compounds. More particularly, the invention relates to aldonamide compounds having a heteroatom (e.g. sulphur, oxygen, nitrogen) in the aliphatic hydrocarbon radical.

### BACKGROUND AND PRIOR ART

An aldonamide is defined as the amide of an aldonic acid (or aldonolactone), and an aldonic acid, in turn, is defined as a sugar substance (eg any cyclic sugar) in which the aldehyde group (generally found at the C₁ position on the sugar) has been replaced by a carboxylic acid which upon drying cyclises to an aldonolactone. Aldonamides may be based on compounds comprising one saccharide unit (eg ribonamides, gluconamide, glucoheptonamide), two saccharide units, (eg lactobionamide or maltobionamide) or they may be based on compounds compromising more than two saccharide units as long as the polysaccharide has a terminal sugar unit with an aldehyde group available for oxidation to a carboxylic acid group.

Most surfactants presently used in personal product and in detergent compositions are based on petrochemicals. Because of increased concern over environmental issues raised by use of such materials and also because of the continually rising costs of petrochemicals, it would be advantageous to develop surfactants which are instead derived from agriculturally grown materials such as carbohydrates. These natural occurring compounds represent a source of renewable raw materials that are synthetically versatile, inexpensive, optically pure and environmentally friendly. In addition, it is most desirable to have surfactants that foam well and are readily soluble in aqueous compositions.

There are a number of prior art references which are directed to N-alkyl gluconamides.

US 2 662 073 (Mehltretter/US Secretary of Agriculture), for example, teaches a gluconamide compound: 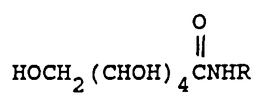 in which R is a hydrocarbon radical selected from aliphatic radicals having 8 to 18 carbons, cycloaliphatic radicals having 8 to 18 carbons and rosin radicals. The compounds are said to be valuable wetting agents for use in the mercerisation of cotton and in the manufacture of viscose yarn. There is clearly no teaching or suggestion of the use of these compounds in detergent or personal product compositions nor is there a teaching or suggestion of using heteroatoms in the hydrocarbon radical (R group) to enhance water solubility.

US 2 721 211 (Buc/GAF Corporation) teaches alkyl formyl phenylene glucamides as solubilising agents for vat dyestuffs. The alkyl, formyl, phenyleneamine hydrocarbon radical (R group) of these compounds are structurally unrelated to the compounds of the invention which contain a hydrocarbon radical interrupted by a heteroatom.

Fieser et al in J Am Chem Soc 78 : 2825 (1956) teach the preparation of a series of N-alkyl arabonamides and N-alkyl gluconamides for use as emulsifiers. In these compounds the attached alkyl R group is C₁₀-C₁₈. The reference teaches that the compounds were insufficiently water-soluble to be useful as emulsifying agents. Furthermore, when oxygen was added to the compound in the form of an additional hydroxyl group on the sugar (ie alkyl glucoheptonamides), the compound had decreased water solubility and poorer emulsifying properties. Thus, the reference suggests that the addition of oxygen in the form of a hydroxyl group in the sugar head group does not enhance water solubility. Also, there is clearly no teaching or suggestion that the addition of oxygen or other heteroatoms in the alkyl radical can enhance solubility.

The fact that monosaccharide aldonamides (eg N-alkyl gluconamides) where the alkyl group is C₁₀ or greater have poor water solubility (i.e., poor emulsifiers) is also recognised in DE 2 321 752 and DE 2 338 087 (Chemische Werke Albert).

Specifically, DE 2 321 752 is directed to N,N-dialkyl polyhydroxyamide compounds having the formula: 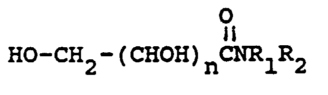 wherein R₁ is an linear or branched aliphatic radical having 4 to 7 carbon atoms, optionally interrupted by oxygen, sulphur, or hydroxyl. Since these compounds are not surface active, they cannot be used as surfactants. DE 2 330 087 broadens the patent application to cover cases where R₁ may be C₈-C₁₀, preferably 8. The principal patent (DE 2 321 752), however, teaches that it is impossible to prepare stable emulsions with N-alkyl aldonamides wherein the alkyl group is N-lauryl (12 carbons), N-cetyl (16 carbons) or N-stearyl (18 carbons).

FR 2 523 962 (Centre Nationale de Recherche) teaches the compound: 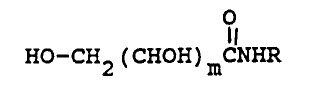 in which m is 2 to 6 and R is a linear or branched alkyl group containing 6 to 18 carbons. The patent further teaches polyoxyethylene, polyoxypropylene or polyglycerol derivatives of the formula. Again, however, there is no teaching that the hydrocarbon radical (R group) may be interrupted by a heteroatom in a way that would make it more soluble relative to an uninterrupted compound.

JP 01 168 653A (Lion) again recognises that the monosaccharide aldonamides of the art (eg N-alkyl gluconamides) do not readily dissolve in water and do not show sufficient surface activity. Again, there is a recognition that such compounds are not readily water soluble and are therefore not useful surfactants.

The Japanese patent publication seeks to address this problem by using N,N-dialkyl polyhydroxy amide compounds where one N-alkyl group is C₆-C₁₈ and the other is C₁-C₄. There is no teaching or suggestion of using a hydrocarbon radical interrupted by a heteroatom to enhance solubility.

US 4 973 473 (Revlon Inc/Schneider et al) teaches skin treatment compositions in which the primary moisturising agent can be a gluconamide. The only example of such ingredient given is the methoxypropyl gluconamide of Example 1 which has the formula: 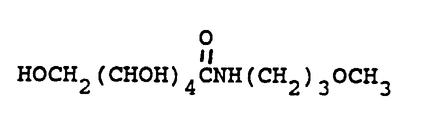 Since these compounds are clearly not surface active, they cannot be used as surfactants. There is no suggestion to utilize alkyl chains greater than methyl and there is clearly no teaching or suggestion that aldonamides containing interrupted long alkyl chains unexpectedly provide much better solubility than their non-interrupted counterparts.

Hoppe-Seyler's Z Physiol Chem 330 : 182 (1963) teaches alkyl gluconyl glycinate compounds as follows: 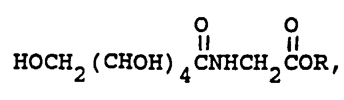 wherein R = C₈ to C₁₀. While this paper does teach monosaccharide aldonamides containing an alkyl group interrupted with an ester functionality, there is no teaching or suggestion that such alkyl groups may be interrupted with, for example, ether oxygen, sulphur or amine, or that the use of such groups will provide superior solubility of the compound relative to the use of other groups. Further, there is clearly no teaching or suggestion, as with any of the above-identified references, that the use of a two-saccharide unit or greater together with an interrupted alkyl group will provide even greater solubility.

Geyer, Chemische Berichte 97 : 2271 (1964), describes the preparation of N-alkanoyl, N-gluconoyl ethylene diamine compounds having the structure (R = C₁₅, C₆) :

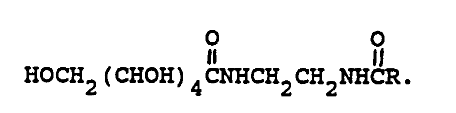

Pfannemuller et al, Chemistry and Physics of Lipids 37 : 227 (1985), describes the preparation of N-alkanoyl-N-methyl-N'-gluconoyl ethylene diamine having the structure (R = C₈):

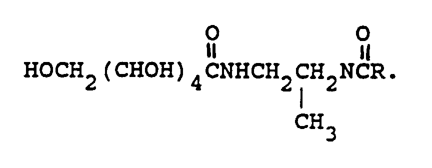

These references teach monosaccharide aldonamides containing an alkyl group that is interrupted with an amide. Again, there is no teaching or suggestion that aldonamides containing alkyl groups interrupted with an ether oxygen, sulphur or amine linkage or that some interrupting groups provide greater solubility than others. Further, there is again clearly no teaching or suggestion that using sugar compounds having two saccharide units or greater (eg lactobionamides) together within an interrupted alkyl group will provide even greater solubility.

In Chemistry Letters 4, 675-678 (1990), "Permeation Characteristics of a Glycolipid Monolayer-Deposited Agar Hydrogel", N Higashi et al disclose the disaccharide aldonamide 2C₁₈-de-MA, containing α-D-glucopyranosyl-D-gluconamide as a hydrophilic head group and two long alkyl chains. There is no disclosure or suggestion of surfactant properties or detergents use.

Finally, there are several references teaching aldobionic compounds (ie compounds having two or more saccharide units).

US 2 752 334 (Walton/National Dairy Research Laboratories) and US 2 785 152 (Jones/National Dairy Products Corporation) teach compounds which are the reaction products of aldobionic acids (eg lactobionic acid) and aldobionolactones with fatty amines or esters of amino acid. The compounds are said to be useful as emulsifiers in food compositions. There is no teaching or suggestion that some lactobionamides have superior properties relative to others and certainly no teaching or suggestion that the use of a heteroatom (eg oxygen, nitrogen or sulphur) in the aliphatic hydrocarbon radical provides superior foaming and solubility relative to lactobionamides having no heteroatom in this radical.

In Williams et al, Archives of Biochem and Biophysics 195(1) : 145-151 (1979), there are described glycolipids prepared by linking aldobionic acids to alkylamine through an amide bond. There is no teaching or suggestion in this reference that the alkyl group of the alkylamine may contain a heteroatom; nor is there any teaching or suggestion that the use of such a heteroatom would provide lactobionamides having enhanced foaming and solubility relative to lactobionamides without a heteroatom.

Accordingly, it would be greatly desirable to find a class of aldonamides which can be successfully used as surfactants in, for example, personal product, dental and detergent compositions, and which also exhibit enhanced foaming and solubility.

EP 550 281A and EP 550 278A (Unilever) are directed to the use of aldobionamide surfactants respectively in personal products and in fabric washing detergent compositions. EP 550 106A (Unilever) discloses a process for the preparation of aldobionamides. EP 551 675A (Unilever) discloses oral hygiene compositions containing aldobionamides as antimicrobial or antiplaque agents. These four documents, which form part of the state of the art by virtue of Article 54(3) EPC, disclose aldobionamides containing heteroatoms.

### DEFINITION OF THE INVENTION

The present invention accordingly provides the use of a monosaccharide aldonamide compound of the general formula I: 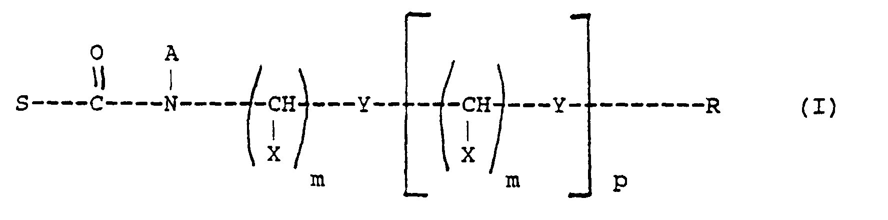 wherein
S represents a monosaccharide unit,
A represents H, a C₁-C₈ hydrocarbon radical, or 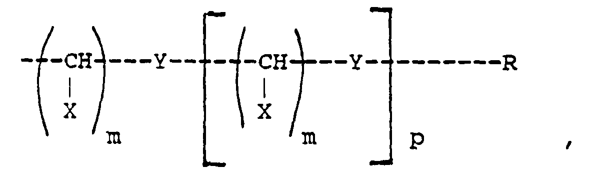
X represents H or a C₁-C₄ alkyl group,
Y represents a group or atom selected from
   -O-, -S-, -NH-, 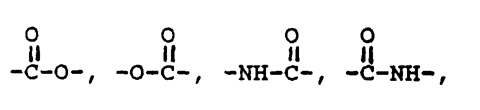
R represents a straight or branched C₈ or above hydrocarbon radical optionally containing a substituted or unsubstituted aromatic or cycloaliphatic radical,
m is an integer from I to 4,
p is 0 or an integer from 1 to 10,
as a surfactant or foaming agent in a surface-active and/or foaming composition.

The invention further provides a detergent composition comprising a detersive and/or foaming amount of a monosaccharide aldonamide compound as defined in the previous paragraph.

The invention further provides, as a novel compound,
a monosaccharide aldonamide compound of the formula Ia, 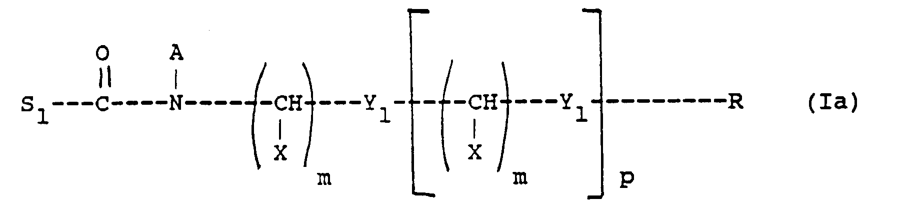 wherein
S₁ represents a monosaccharide unit of the formula 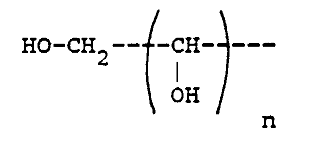 wherein n represents an integer of from 1 to 6,
A represents H, a C₁-C₈ hydrocarbon radical, or 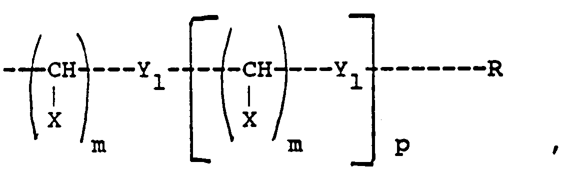
X represents H or a C₁-C₄ alkyl group,
Y₁ represents -O-, -S- or -NH-,
R represents a straight or branched C₈ or above hydrocarbon radical optionally containing a substituted or unsubstituted aromatic or cycloaliphatic radical,
m is an integer from 1 to 4,
p is 0 or an integer from 1 to 10.

### DETAILED DESCRIPTION OF THE INVENTION

The present invention relates to a class of environmentally friendly nonionic surfactants known as aldonamides. In particular, the invention relates to specific classes of aldonamide surfactants having enhanced foaming and solubility characteristics relative to other aldonamides.

The invention encompasses the use as surfactants and foaming agents of a defined class of aldonamide surfactants which have superior foaming and solubility characteristics relative to other aldonamides (eg both monosaccharide aldonamides and disaccharide aldobionamides).

Within that defined class, a specific embodiment of the invention relates to a novel subclass of aldonamides, and these are also claimed as novel compounds.

In general, an aldonamide is defined as the amide of an aldonic acid or aldonolactone and an aldonic acid, in turn, is a sugar substance (eg any cyclic sugar) wherein the aldehyde group (generally found at the C₁ position of the sugar) has been replaced by a carboxylic acid, which cyclises to an aldonolactone upon drying.

Since an aldonamide is defined by the terminal sugar, an aldonamide may be based on compounds comprising one saccharide unit (eg ribonamides, gluconamides, glucoheptonamides), two saccharide units (eg lactobionamides or maltobionamides) or they may be based on compounds comprising more than two saccharide units, as long as the terminal sugar in the polysaccharide has an aldehyde group which may be converted to an aldonic acid or aldonolactone.

While not wishing to be bound by theory, it is believed that the amphiphilic nature of alkyl aldonamides and aldobionamides cause them to concentrate at the surface interface thereby reducing the free energy of the system.

However, when all available interfaces are saturated, the overall energy reduction may be through precipitation or soluble micelle formation. In general, the preferred interfacial phenomenon is micellisation since detergency and solubilisation of alkyl aldonamides in detergents and personal products depend on the existence of these aggregates in solution. It is also favourable for micellisation to occur at low Krafft temperatures, since alkyl aldonamides that lack a heteroatom in the hydrocarbon chain have an unfavourable heat of hydration and often precipitate out of solution rendering them ineffective.

We have found that the addition of heteroatoms such as oxygen, nitrogen or sulphur unexpectedly reduced the packing constraints of these materials in the solid state. The net result is a more favorable heat of hydration, a lower Krafft point, enhanced rate of micellisation and superior foaming.

Also closer observation will reveal that compounds of this invention also unexpectedly allow the introduction of the same or greater alkyl chain length (differing only by the presence of the heteroatom) without simultaneously sacrificing foaming and solubility characteristics.

The aldonamides used in the composition of the invention have solubilising and foam stabilising properties which are superior to aldonamides having no heteroatom in the hydrocarbon radical.

### Preferred compounds used in the invention

In all compounds that are used in accordance with the invention, the A group is preferably hydrdgen although it may be a C₁ to C₈ straight chain or branched chain, saturated or unsaturated hydrocarbon.

When A is a C₁ to C₈ hydrocarbon radical, it may be straight chain or branched, saturated or unsaturated aliphatic; aromatic; mixed aromatic/aliphatic.

The A group may also be interrupted by a heteroatom and may have the same structure as the other monosaccharide group attached to the nitrogen.

If this A group is an aliphatic radical, suitable aliphatic hydrocarbon radicals include saturated and unsaturated radicals including but not limited to methyl, ethyl, amyl, hexyl, heptyl, nonyl, decyl, undecyl, dodecyl, tridecyl, tetradecyl, pentadecyl, hexadecyl, heptadecyl, and octadecyl, and allyl, undecenyl, oleyl, linoleyl, linolenyl, propenyl, and heptenyl.

If the A group is an aromatic group, the aromatic radical may be, for example, benzyl.

Suitable mixed aliphatic aromatic radicals are exemplified by benzyl, phenyl ethyl, and vinyl benzyl.

R may be a straight or branched chain, saturated or unsaturated hydrocarbon backbone which may contain a substituted or unsubstituted aromatic radical or cycloaliphatic radical, wherein the hydrocarbon backbone comprises 8 or more carbon atoms.

### Novel compounds of the invention

In one embodiment, the invention relates to a class of single saccharide unit compounds of the formula Ia above (eg gluconamides) having an ether group, sulphur atom or amine in the aliphatic hydrocarbon radical.

### The monosaccharide aldonamides

These compounds may be represented by the general formula below, which is equivalent to formula Ia given above: 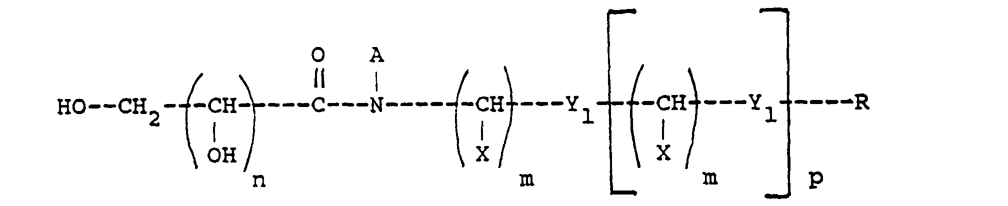 wherein n, A, m, Y₁, p and R have the meanings given previously.

Preferred examples of monosaccharide aldonamides in accordance with the invention include C₈ to C₁₅ oxypropyl D-gluconamides and C₁₆ to C₂₁ aminopropyl D-gluconamides.

A specific example is dodecyl oxypropyl gluconamide having the structure above wherein n = 4, X = hydrogen, m = 3, Y = oxygen, p = 0, A = hydrogen, and R = C₁₂ straight chain alkyl.

### DETERGENT COMPOSITIONS

The novel detergent components of the present invention may be incorporated in detergent compositions of all physical types, for example, powders, liquids, gels and solid bars. They may, for example, be incorporated at low levels to enhance the foam of other detergent-active compounds, or in more substantial amounts as surfactants in their own right.

These compositions may generally contain other detergent-active compounds, detergency builders, bleaching components and other active ingredients to enhance performance and properties.

Other detergent-active compounds (surfactants) present may be chosen from soap and non-soap anionic, cationic, nonionic, amphoteric and zwitterionic detergent-active compounds, and mixtures thereof. Many suitable detergent-active compounds are available and are fully described in the literature, for example, in "Surface-Active Agents and Detergents", Volumes I and II, by Schwartz, Perry and Berch.

The preferred detergent-active compounds that can be used are soaps and synthetic non-soap anionic and nonionic compounds.

Detergent compositions of the invention may also, depending on the purpose for which they have been formulated, contain various ancillary ingredients. For example, fabric washing compositions may contain detergency builders; bleach components; sodium carbonate; sodium silicate; antiredeposition agents such as cellulosic polymers; fluorescers; inorganic salts such as sodium sulphate; proteolytic and lipolytic enzymes; dyes; coloured speckles; perfumes; fabric softening compounds. This list is not intended to be exhaustive.

The compounds of the invention are also suitable for other applications in which surfactant and/or foaming properties are useful, for example shampoos and personal washing, personal care and dental care compositions.

### EXAMPLES

The invention is set forth in greater detail in the examples which follow below. These examples are intended to be illustrative only and are not intended to be limiting in any way.

### Example 1

### Preparation of coco D-gluconamide (used for comparative purposes)

A 5-litre four-necked round bottom flask equipped with a condenser, addition funnel, thermometer and mechanical stirrer was charged with D-glucono-1,5-lactone (480 g, 2.69 moles) and methanol (2752 g, for 27% total solids). The suspension was heated to 40-50°C for 15 minutes and the heating mantle removed. Cocoamine (538 g, 2.69 moles) containing methanol (80 ml) was added dropwise over 1/2 hour. The reaction mixture was allowed to cool to room temperature (about 21°C) followed by stirring overnight to allow complete crystallisation. The white product was filtered, washed with methanol (3 x 500 ml) and dried under vacuum at 40-45°C giving 947 g (93% yield) of coco D-gluconamide with a melting point of 147-148°C.

### Analysis of monosaccharide aldonamides by gas chromatography

Gas chromatography was found to be a convenient method for the examination of monosaccharide aldonamides. The method of persilylation with hexamethyldisilazane (HMDS) and trimethylchlorosilane (TMCS) in pyridine is the simplest way for producing sufficiently stable and volatile derivatives for analysis. The mixture of both agents are more reactive than either reagent alone, and the by-products combine to form neutral ammonium chloride or pyridine hydrochloride.

The purity of several monosaccharide aldonamides were determined and found to be 97-99.9%. All products were well separated from starting materials, however aldonamides with alkyl groups containing eighteen carbons or more were not volatile enough for analysis.

Approximately 7-10 mg of monosaccharide aldonamide was treated with 1 ml of sil-prep reagent (pyridine: HMDS:TMS=9:3:1) in a 1 dram stoppered vial containing a magnetic stirring bar. The mixture was stirred vigorously at room temperature for 1 hour or longer prior to chromatography. The solution became cloudy owing to precipitation of NH₄Cl and pyridine HCl which was filtered through a Cameo (Trade Mark) II 25 mm filter. From 1-1.1 microlitres of the resulting mixture was injected into the gas chromatograph.

All gas chromatography was conducted on a Hewlett Packard (Trade Mark) 5890 Series II Gas Chromatograph. All sample components were detected by a flame ionisation detector using a split ratio of 100:1 and separated on a crosslinked 5% phenylmethyl silicone capillary column 25 m x 0.32 mm x 0.53 micrometres. The carrier gas was helium at 1 ml/min and the temperature program was 3 min at 140°C then 30°C /min to 250°C for 75 min.

### Examples 2-18

The alkyl aldonamides 2-18 in Table 1 were prepared as in Example 1.

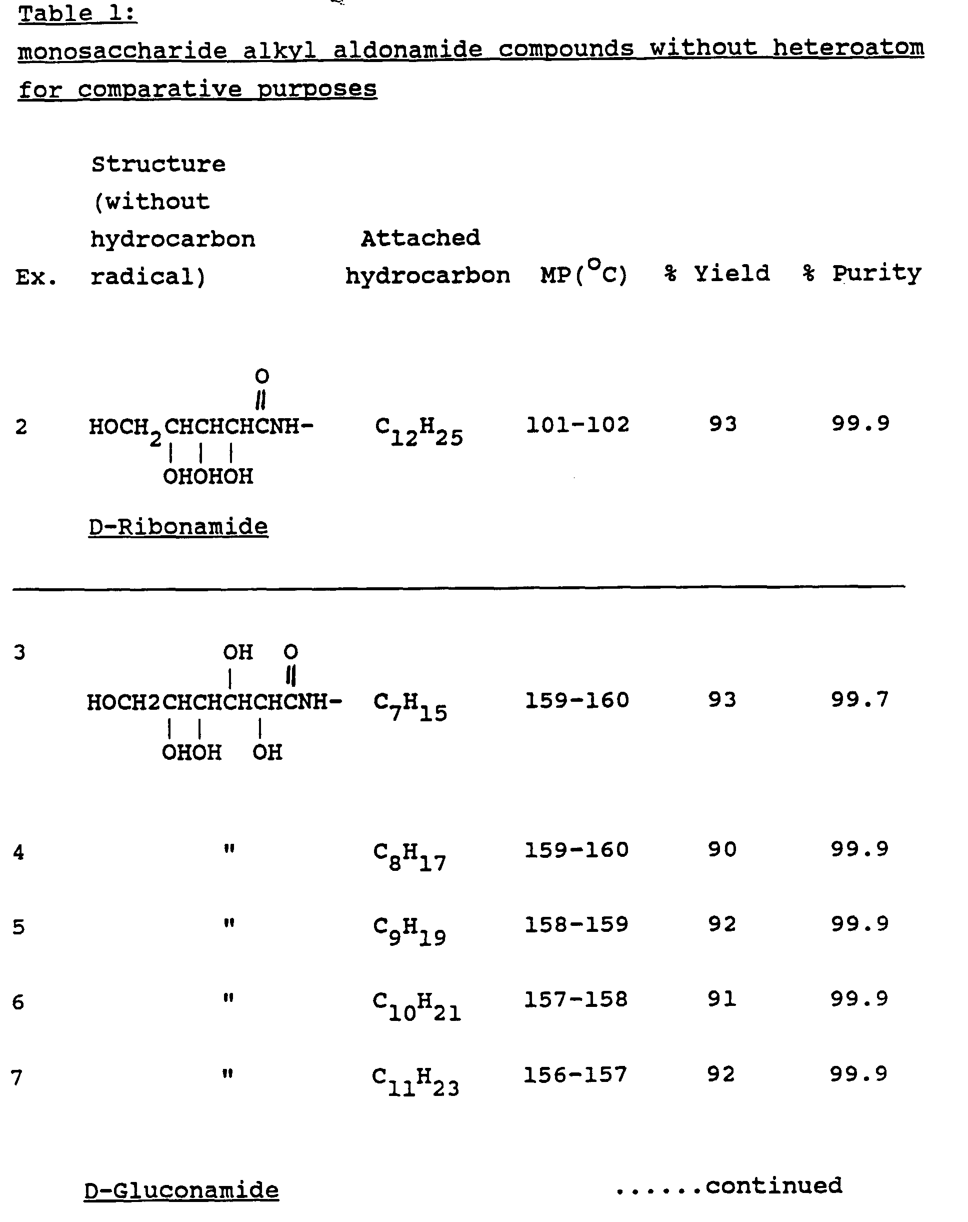

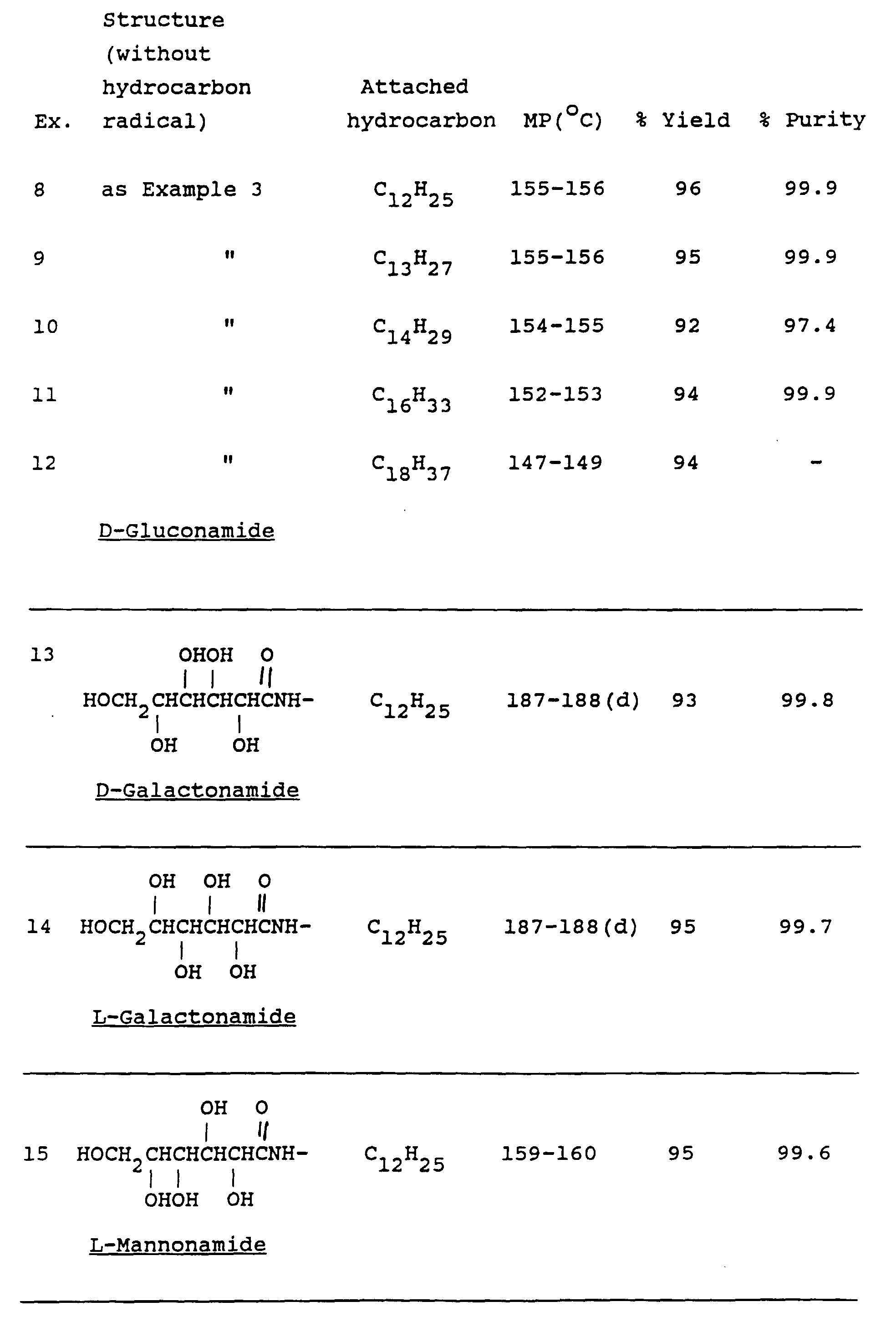

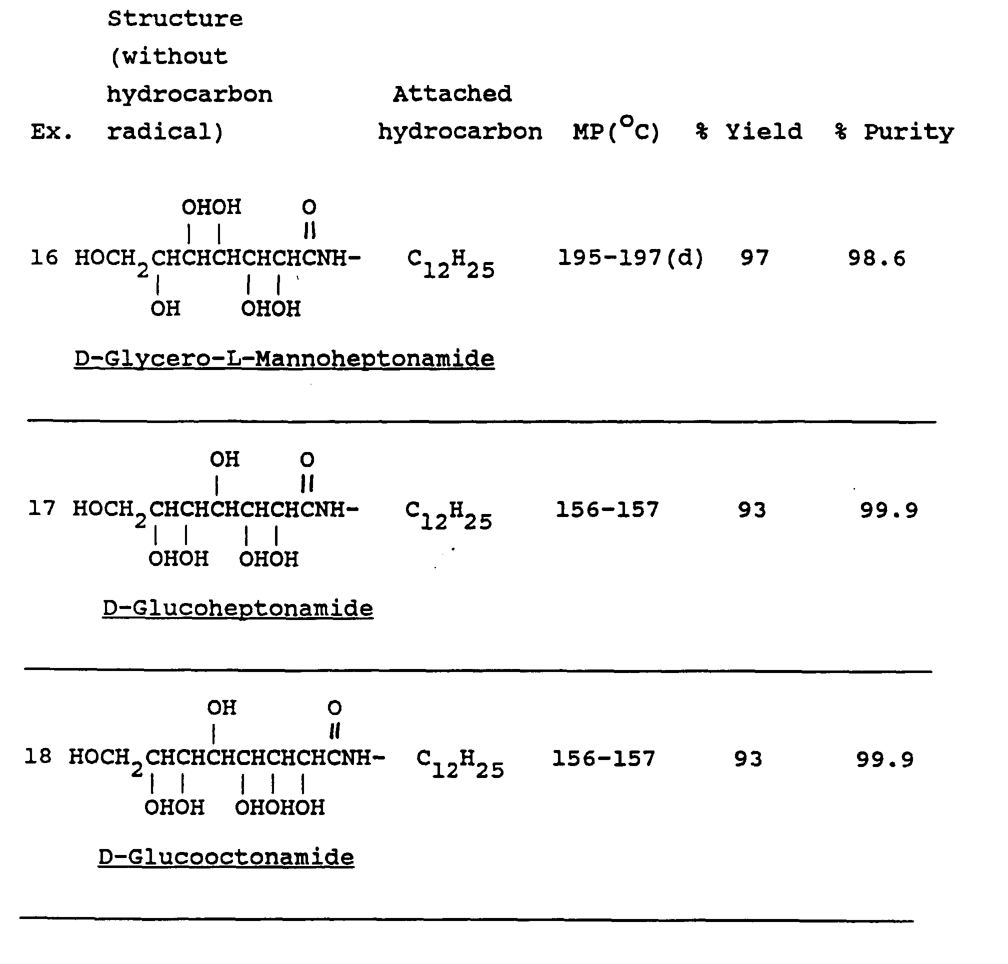

### Example 19

### Preparation of octyl/decylolyprocyl D-ribonamide

A 250 ml four-necked round bottom flask equipped with a condenser, additional funnel, thermometer and mechanical stirrer was charged with ribono-1,4-lactone (10 g, 0.068 mole) and methanol (37 g, for 40% solids). The suspension was heated to 40-50°C for 15 minutes and heating mantle removed. Octyl/decyloxypropylamine (14.6 g, 0.68 mole) was added dropwise over 1/2 hour and the reaction mixture stirred for 6 hours. The white product was filtered, washed with cold acetone (3 x 10 ml) and dried under vacuum at 40-45°C giving 14 g (57% yield) of octyl/decyloxypropyl D-ribonamide with a melting point of 71-72°C and 98.7% purity (C₈/C₁₀: 62.8%/35.9%).

### Examples 20-28 (oxygen heteroatom)

The monosaccharide alkyloxypropyl aldonamides 20-28 in Table 2 were prepared as in Example 19.

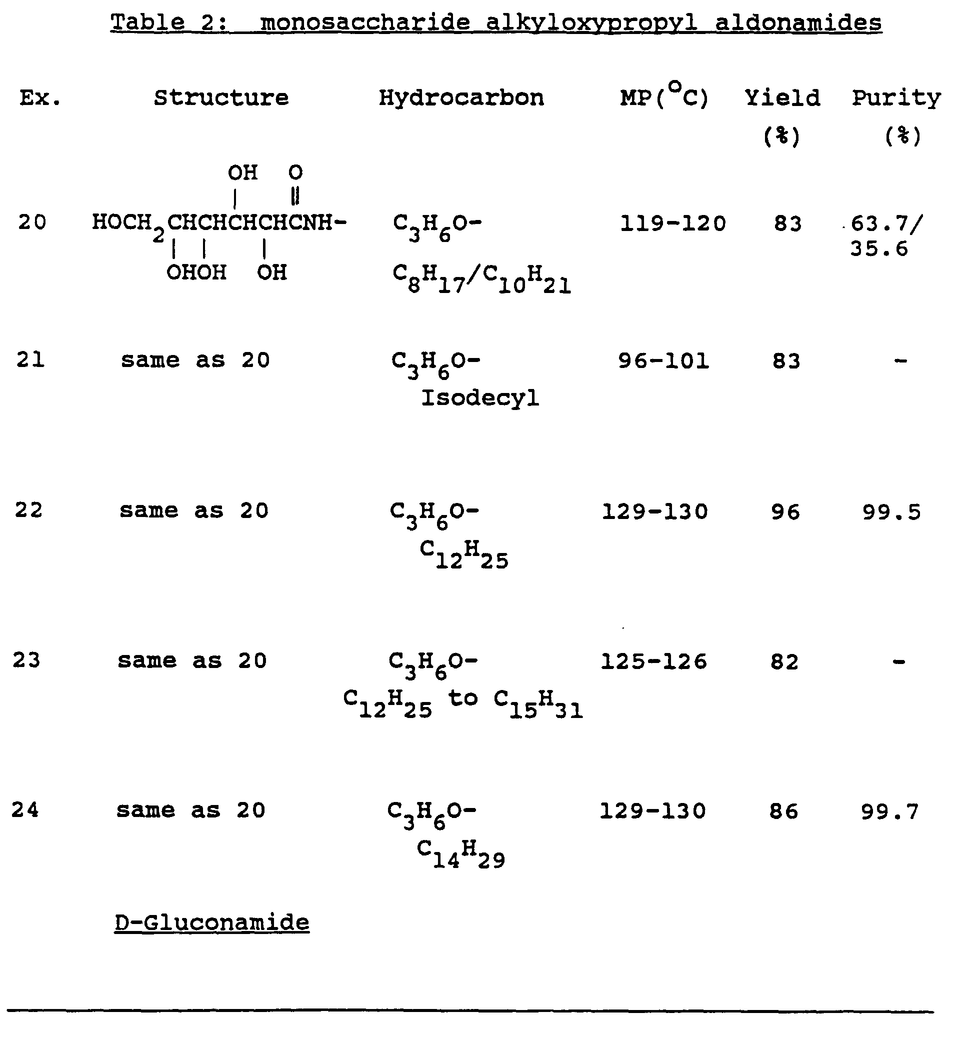

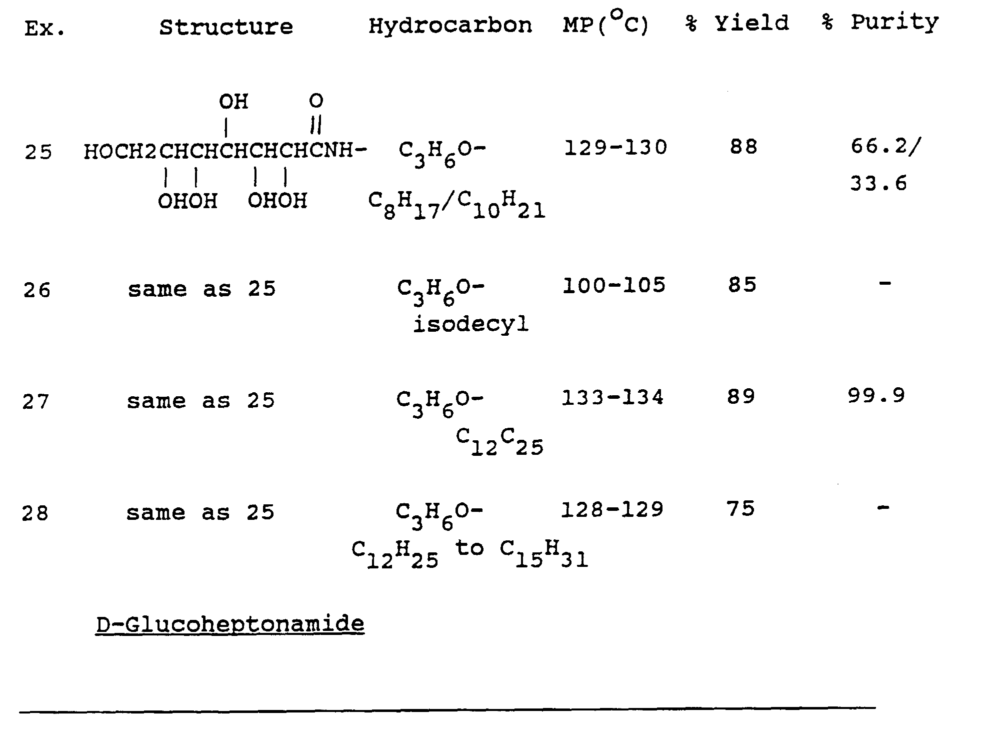

### Example 29 (nitrogen heteroatom) Preparation of cocoaminopropyl D-gluconamide

A l-litre round-bottomed flask equipped with a condenser, addition funnel, thermometer and mechanical stirrer was charged with D-glucono-1,5-lactone (100 g, 0.56 mole) and methanol (208 g for 55% total solids). The suspension was heated to 40°C over 15 minutes and the heating mantle removed. Cocoaminopropylamine (153.8 g, 0.56 mole) was added dropwise over 15 minutes with rapid stirring. The reaction mixture was cooled and placed in a refrigerator at 0°C overnight. The white product was filtered, washed with cold isopropanol (3 x 100 ml) and dried under high vacuum at 35°C giving 206 g (81% yield) of cocoaminopropyl D-glucononamide with a melting point of 109-111°C.

### Example 30 (nitrogen heteroatom)

Soyaaminopropyl D-gluconamide was prepared as in Example 29.

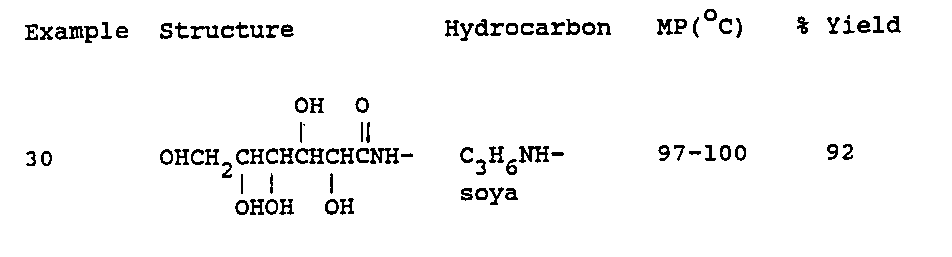

### Example 31 (nitrogen heteroatom) Preparation of oleylaminopropyl D-gluconamide

A 500 ml round-bottomed flask equipped with a condenser, addition funnel, thermometer and mechanical stirrer was charged with D-glucono-1,5-lactone (25 g, 0.14 mole) and methanol (31 g, 70% total solids). The suspension was heated to 40°C for 15 minutes and the heating mantle removed. Oleylaminopropylamine (47.72 g, 0.14 mole) was added dropwise over 10 minutes and the reaction stirred for 6 hours. Acetone (300 ml) was added and the flask placed in a refrigerator at 0°C overnight. The white product was filtered, washed with cold acetone (3 x 50 g) and dried under high vacuum at 35°C giving 65 g (89% yield) of oleylaminopropyl D-gluconamide with a melting point of 100-103°C.

### Examples 32-33 (oxygen and nitrogen heteroatoms)

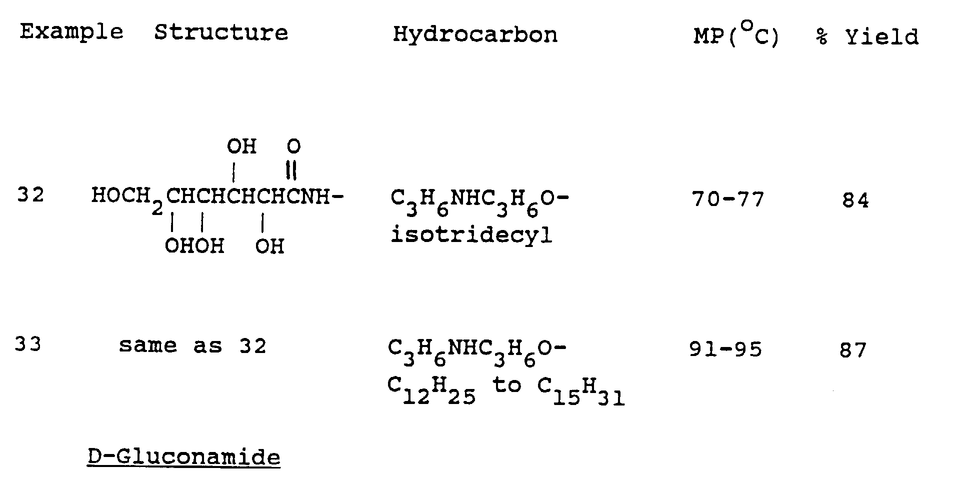

In each of Examples 20-33, it should be appreciated, to the extent it is not clear, that the hydrocarbon radical is all one radical with the portion of the radical on any line attached to the portion immediately above it. For example, in Example 21, the radical is C₃H₆O-isodecyl. Also a slash represents a mixture of radicals. Thus, Example 20 represents a mixture of C₃H₆OC₈H₁₇ and C₃H₆OC₁₀H₂₁ radicals.

### Example 34: Krafft Points

The temperature at and above which surfactants begin to form micelles instead of precipitates is referred to as Krafft Point (Tₖ) and at this temperature the solubility of a surfactant becomes equal to its critical micelle concentration or CMC (numerical value at which micelles are formed).

The appearance and development of micelles are important since detergency and solubilisation of surfactants in dishwashing liquids, shampoos, detergents, etc depend on the formation of these aggregates in solution.

The Krafft point was measured by preparing 650 ml of a 0.1% or 1.0% by weight dispersion of aldonamide in water. If the surfactant was soluble at room temperature, the solution was slowly cooled to 0°C. If the surfactant did not precipitate out of solution, its Krafft point was considered to be <0°C. If it precipitated out of solution, the temperature at which precipitation occurred was taken as the Krafft point.

If the surfactant was insoluble at room temperature, the dispersion was slowly heated until the solution became homogeneous. It was then slowly cooled until precipitation occurred. The temperature at which the surfactant precipitated out of solution was taken as the Krafft point.

The results are set out below under Examples 36 - 38.

### Example 35: Foam Height

Foam is an important attribute in many consumer products. It is one of the dominant factors that determines the commercial value of products such as shampoo, soap, etc. Also, acceptability of many consumer products is closely related to the quality and texture of the foam they produce (psychological aspect).

Since most foaming data on surfactants is typically obtained by the Ross-Miles method (Ross J and Miles G D, Am Soc for Testing Material Method D1173-53, Philadelphia, PA. (1953); Oil & Soap (1958) 62 : 1260) the foaming ability of these surfactants was also assessed using this method.

In the Ross-Miles method, 200 ml of a solution of surfactant contained in a pipette of specified dimensions with a 2.9 mm internal diameter orifice is allowed to fall 90 cm onto 50 ml of the same solution contained in a cylindrical vessel maintained at a given temperature (often 60°C) by means of a water jacket. The height of the foam produced in the cylindrical vessel is read immediately after all the solution has run out of the pipette (initial foam height) and then again after a given amount of time (generally, 5 minutes).

Using this method, the foam production (initial foam height in mm) and foam stability (final foam height after 10 minutes in mm) were measured at 0.1% aldonamide concentration 40°C and 0 ppm (parts per million) hardness. Aldonamides that were not soluble at 40°C were measured 5-10°C above their Krafft point.

In order to show the unexpected enhancement in solubility and foam stabilisation of the compounds of the invention relative to similar aldonamide compounds having no heteroatom in the attached aliphatic group, applicants compared a series of alkyl aldonamides and aldobionamides lacking a heteroatom in the hydrocarbon radical to those containing a heteroatom in the hydrocarbon radical.

The results are set out below under Examples 36 - 38.

### Example 36

| Monosaccharide aldonamides containing four hydroxyl groups | | | | | |
|---|---|---|---|---|---|
| Entry | Compound | Hydrocarbon chain length (average) | Foam height (mm) | | Krafft point (°C) |
| | | | initial | final | |
| A | C₁₂ ribonamide (comparative) | 12 | 184 | 103 | 54 |
| B | C₈/C₁₀ oxypropyl D-ribonamide | 11.5 | 217 | 200 | 10 |

This example clearly shows that when a heteroatom is added, foaming is enhanced and solubility increased.

### Example 37

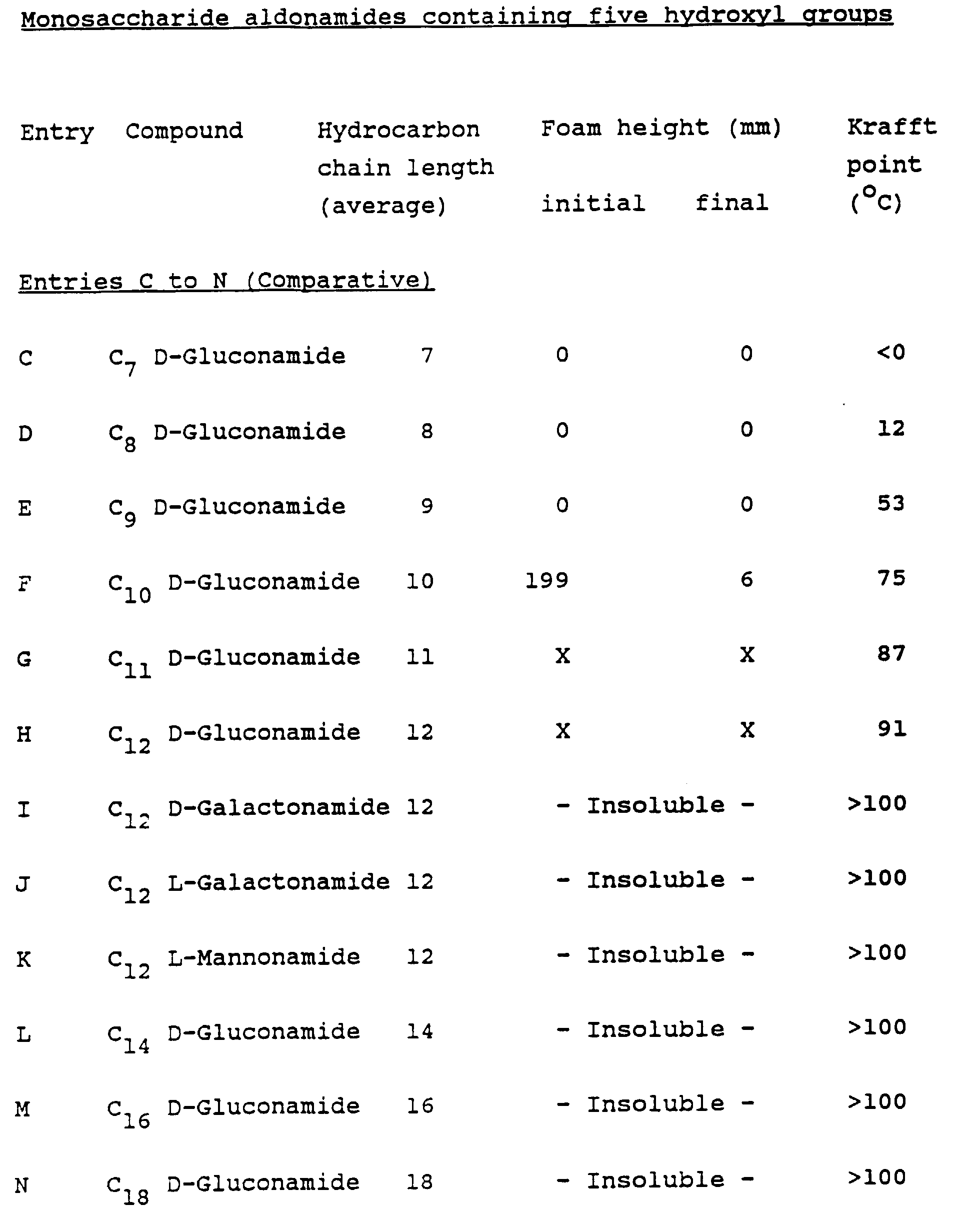

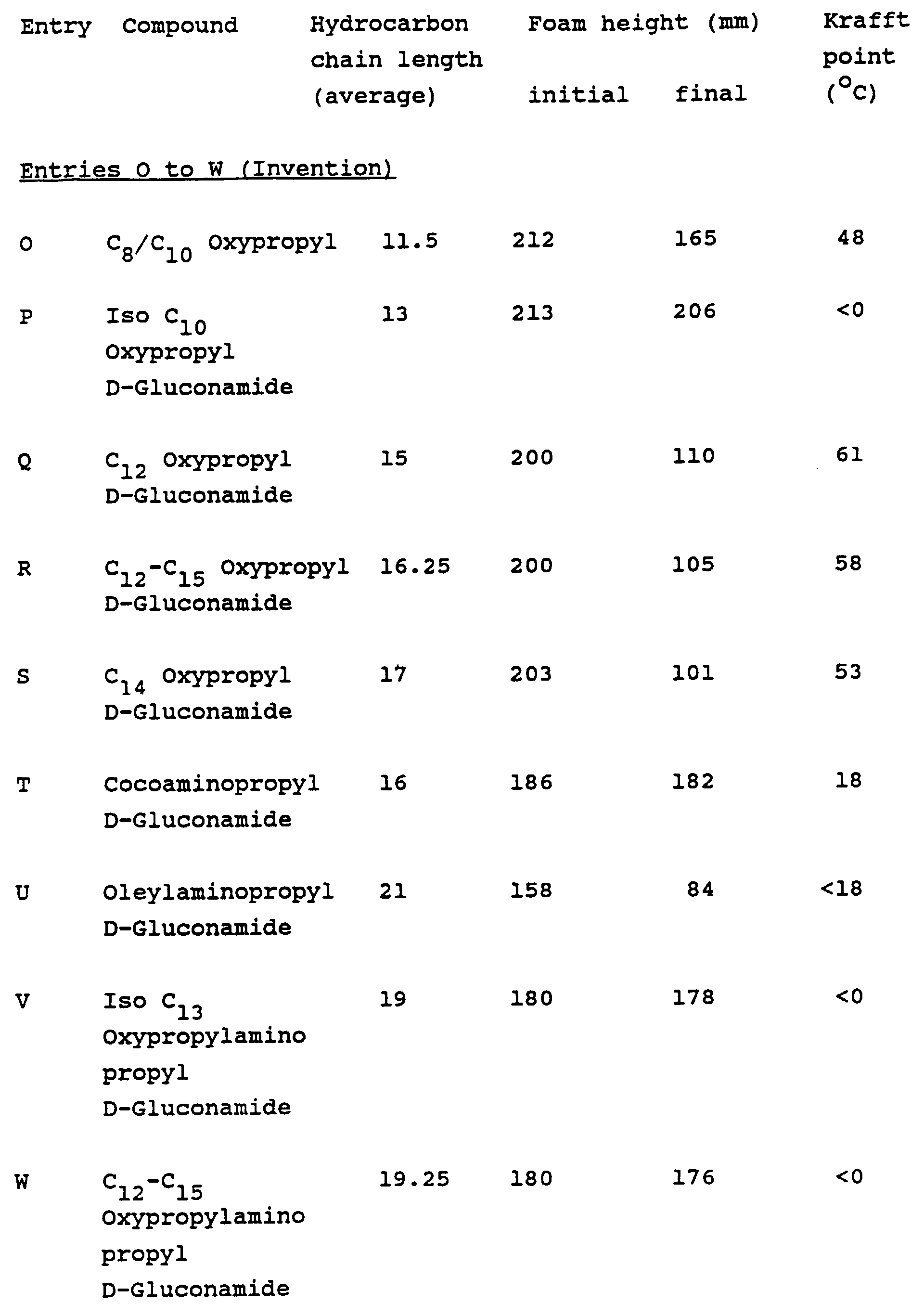

X in the Table indicates that the foam height of these high Krafft point materials could not be measured.

Entries C-N are comparative and O-W are compounds of the invention. The best comparisons are based on average number of hydrocarbons. From the above table, it can be seen that compounds without heteroatoms become increasingly insoluble in water resulting in poor or no foaming.

By contrast, when a heteroatom is present, enhanced foaming and reduced Krafft point can be seen in all cases, even entry U which contains 21 carbon atoms.

Also, it can be seen that when more than one heteroatom is present (entries V and W), Krafft points are significantly lower (ie less than 0) indicating enhanced solubility for compounds that have a longer than average hydrocarbon chain length which otherwise would be normally insoluble.

### Example 38

### Monosaccharide aldonamides containing six hydroxyl groups

This example shows that merely increasing hydrophilicity of the sugar (by hydroxyl group addition) does not enhance solubility (comparative entries X, Y & Z). However, when a heteroatom is introduced into the hydrocarbon radical Krafft points are reduced and foaming enhanced (entries AA-DD).

### Example 38

| Monosaccharide aldonamides containing six hydroxyl groups | | | | | |
|---|---|---|---|---|---|
| Entry | Compound | Hydrocarbon chain length (average) | Foam height (mm) | | Krafft point (°C) |
| | | | initial | final | |
| Entries X to Z (comparative) | | | | | |
| X | C₁₂ D-Glycero-L-Mannoheptonamide | 12 | -Insoluble- | | >100 |
| Y | C₁₂ D-Glucoheptonamide | 12 | X | X | 91 |
| Z | C₁₂ D-Glucooctonamide | 12 | X | X | 86 |

| Entries AA to DD (invention) | | | | | |
|---|---|---|---|---|---|
| AA | C₈/C₁₀ Oxypropyl D-Glucoheptonamide | 11.5 | 221 | 90 | 60 |
| BB | Iso C₁₀ Oxypropyl D-Glucoheptonamide | 13 | 215 | 204 | 18 |
| CC | C₁₂ Oxypropyl D-Glucoheptonamide | 15 | 245 | 80 | 73 |
| DD | C₁₂ -C₁₅ Oxypropyl D-Glucoheptonamide | 16.25 | 239 | 97 | 68 |

### Discussion of Examples 36 - 38

The data clearly show that alkyl gluconamides lacking a heteroatom in the hydrocarbon chain (E-H, L-N) are inferior foamers and have higher Krafft points. While not wishing to be bound by theory, it is believed that these compounds pack closely in the solid state through strong amide/hydroxyl hydrogen bonding and hydrocarbon Van der Waal forces. The net results is an unfavourable heat of hydration, high Krafft point, low or no water solubility and poor foaming profile. Changing the stereochemistry (I-K) or increasing the hydrophilicity (X-Z) of the sugar head group (by hydroxyl group addition) results in little improvement. However, monosaccharide alkyl aldonamides that contain a heteroatom such as oxygen (B,O-S, AA-DD) or nitrogen (T, U) on both (V, W) in the hydrocarbon chain are believed to pack more favourably in the solid state thereby resulting in a low Krafft point, increased water solubility and superior foaming profile.

Also, closer comparison reveals that monosaccharide aldonamides containing a heteroatom in the hydrocarbon chain unexpectedly allow the introduction of the same or greater alkyl chain length without sacrificing foaming and solubility characteristics. (Compare the average number of hydrocarbons of L-N to Q-W and Y to AA-DD).

Disaccharide alkyl lactobionamides (EE-GG) tend to have reasonable Krafft points and foaming profiles. However, the addition of a heteroatom such as oxygen in the form of an ester (HH, II) or ether (JJ, KK) in the hydrocarbon chain results in a extremely low Krafft points (<0°C), increased water solubility and enhanced foaming.

## Claims

1. Use of a monosaccharide aldonamide compound of the general formula I: 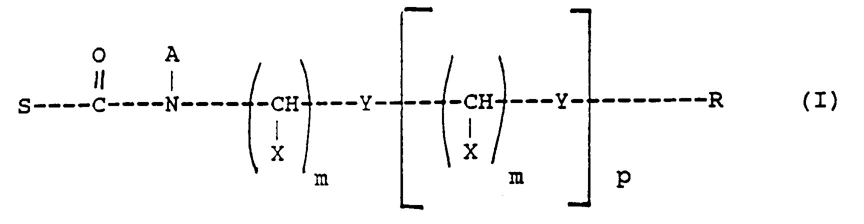 wherein S represents a monosaccharide unit,
A represents H, a C₁-C₈ hydrocarbon radical, or 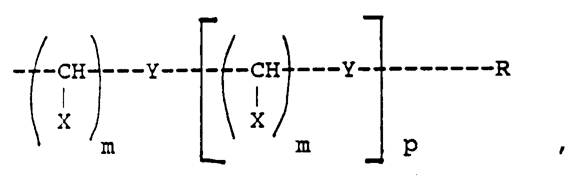
X represents H or a C₁-C₄ alkyl group,
Y represents a group or atom selected from
-O-, -S-, -NH-, 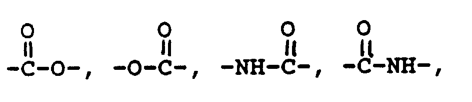
R represents a straight or branched C₈ or above hydrocarbon radical optionally containing a substituted or unsubstituted aromatic or cycloaliphatic radical,
m is an integer from 1 to 4,
p is 0 or an integer from 1 to 10,
as a surfactant or foaming agent in a surface-active and/or foaming composition.

2. Use of a compound as claimed in claim 1, wherein S represents a monosaccharide unit of the formula: 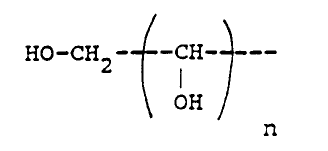 wherein n represents an integer of from 1 to 6.

3. Use of a compound as claimed in claim 2, wherein n is 4.

4. Use of a compound as claimed in claim 2, wherein Y represents O, S or NH.

5. Use of a compound as claimed in any preceding claim wherein X represents a hydrogen atom.

6. Use of a compound as claimed in any preceding claim wherein m is 3.

7. Use of a compound as claimed in any preceding claim wherein Y represents an oxygen atom.

8. Use of a compound as claimed in any preceding claim wherein p is zero.

9. Use of a compound as claimed in any preceding claim wherein A represents a hydrogen atom.

10. Use of a compound as claimed in any preceding claim wherein R represents a C₈ to C₂₄ alkyl group.

11. Use of a compound as claimed in claim 10 wherein R represents a C₁₀ to C₁₄ alkyl group.

12. Use of a compound as claimed in claim 1 which is a C₈ to C₁₅ oxypropyl D-gluconamide.

13. Use of a compound in claimed in claim 1 which is a C₁₆ to C₂₁ aminopropyl D-gluconamide.

14. A detergent composition comprising a detersive and/or foaming amount of a compound of the formula I as defined in claim 1.

15. A monosaccharide aldonamide compound of the formula Ia, 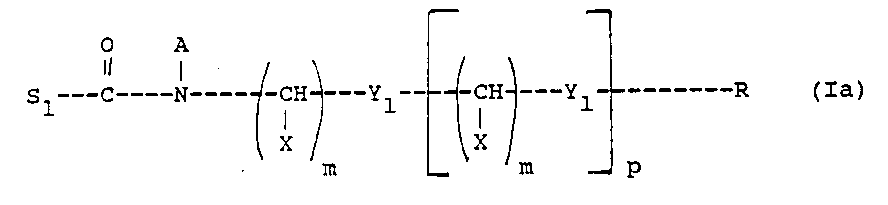 wherein S₁ represents a monosaccharide unit of the formula 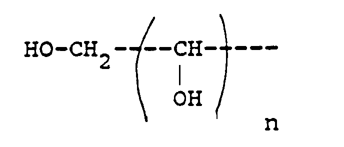 wherein n represents an integer of from 1 to 6,
A represents H, a C₁-C₈ hydrocarbon radical, or 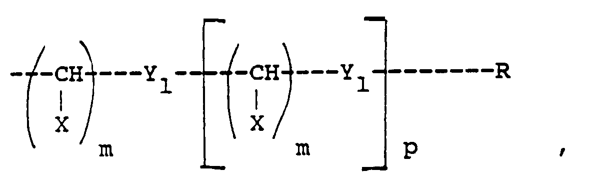
X represents H or a C₁-C₄ alkyl group,
Y₁ represents -O-, -S- or -NH-,
R represents a straight or branched C₈ or above hydrocarbon radical optionally containing a substituted or unsubstituted aromatic or cycloaliphatic radical,
m is an integer from 1 to 4,
p is 0 or an integer from 1 to 10.

## Patentansprüche

1. Verwendung einer Monosaccharid-Aldonamid-Verbindung der allgemeinen Formel I: 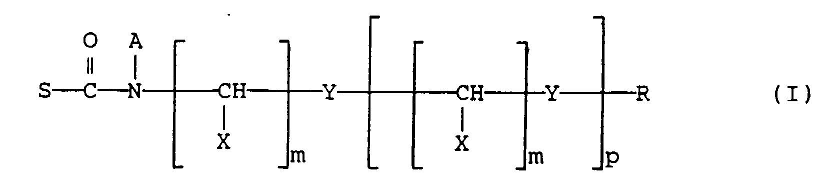 worin S eine Monosaccharid-Einheit bedeutet,
A H, einen C₁₋₈-Kohlenwasserstoffrest, oder 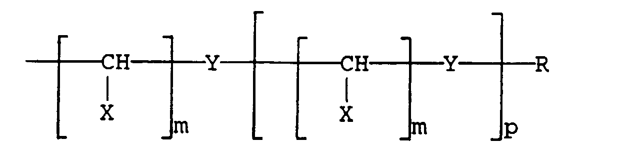 bedeutet,
X H oder eine C₁₋₄-Alkylgruppe bedeutet,
Y eine Gruppe oder ein Atom bedeutet, ausgewählt aus
-O-, -S-, -NH-, 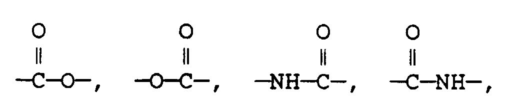
R einen geradkettigen oder verzweigtkettigen C₈- oder darüberliegenden Kohlenwasserstoffrest bedeutet, gegebenenfalls enthaltend einen substituierten oder nichtsubstituierten aromatischen oder cycloaliphatischen Rest,
m eine ganze Zahl mit einem Wert von 1 bis 4 ist,
p 0 oder eine ganze Zahl mit einem Wert von 1 bis 10 bedeutet,
als eine grenzflächenaktive Substanz oder als ein Schaumbildner in einer oberflächenaktiven und/oder schäumenden Zusammensetzung.

2. Verwendung einer Verbindung wie in Anspruch 1 beansprucht, worin S eine Monosaccharid-Einheit der Formel 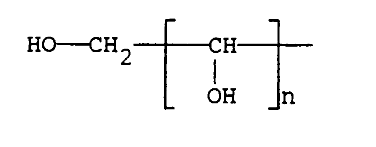 bedeutet, worin n eine ganze Zahl mit einem Wert von 1 bis 6 ist.

3. Verwendung einer Verbindung wie in Anspruch 2 beansprucht, worin n den Wert 4 hat.

4. Verwendung einer Verbindung wie in Anspruch 2 beansprucht, worin Y O, S oder NH bedeutet.

5. Verwendung einer Verbindung nach einem der vorstehenden Ansprüche, worin X ein Wasserstoffatom bedeutet.

6. Verwendung einer Verbindung nach einem der vorstehenden Ansprüche, worin m den Wert 3 hat.

7. Verwendung einer Verbindung nach einem der vorstehenden Ansprüche, worin Y ein Sauerstoffatom bedeutet.

8. Verwendung einer Verbindung nach einem der vorstehenden Ansprüche, worin p den Wert Null hat.

9. Verwendung einer Verbindung nach einem der vorstehenden Ansprüche, worin A ein Wasserstoffatom bedeutet.

10. Verwendung einer Verbindung nach einem der vorstehenden Ansprüche, worin R eine C₈₋₂₄-Alkylgruppe bedeutet.

11. Verwendung einer Verbindung nach Anspruch 10, worin R eine C₁₀₋₁₄-Alkylgruppe ist.

12. Verwendung einer Verbindung nach Anspruch 1, welche ein C₈₋₁₅-Oxypropyi-D-gluconamid ist.

13. Verwendung einer Verbindung nach Anspruch 1, welche ein C₁₆₋₂₁-Aminopropyl-D-gluconamid ist.

14. Eine Detergens-Zusammensetzung, enthaltend eine reinigende und/oder schäumende Menge einer Verbindung der Formel I, wie in Anspruch 1 definiert.

15. Eine Monosaccharid-Aldonamid-Verbindung der Formel Ia 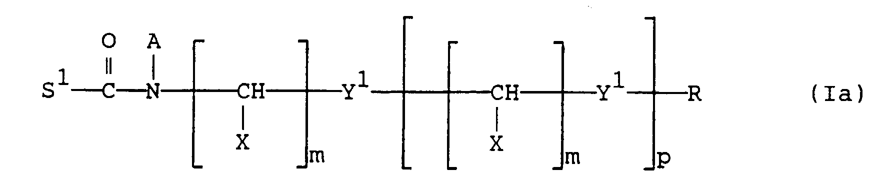 worin S¹ eine Monosaccharid-Einheit der Formel 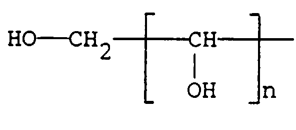 bedeutet, worin n eine ganze Zahl mit einem Wert von 1 bis 6 ist,
A H, einen C₁₋₈-Kohlenwasserstoffrest, oder 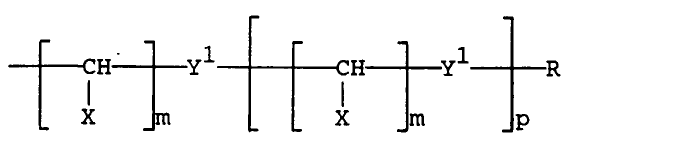 bedeutet,
X H oder eine C₁₋₄-Alkylgruppe bedeutet,
Y¹ -O-, -S- oder -NH- bedeutet,
R einen geradkettigen oder verzweigtkettigen C₈- oder darüberliegenden Kohlenwasserstoffrest bedeutet, gegebenenfalls enthaltend einen substituierten oder nichtsubstituierten aromatischen oder cycloaliphatischen Rest,
m eine ganze Zahl mit einem Wert von 1 bis 4 ist,
p 0 oder eine ganze Zahl mit einem Wert von 1 bis 10 ist.

## Revendications

1. Utilisation d'un composé aldonamide monosaccharide ayant la formule générale : 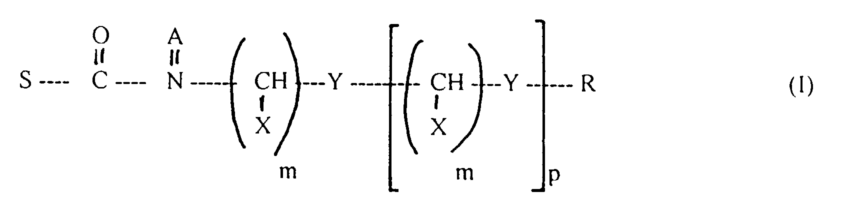 dans laquelle :
S représente une unité monosaccharide
A représente H, un radical hydrocarbure en C₁ - C₈, ou 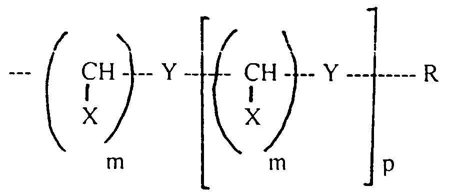
X représente H ou un groupe alkyle en C₁ - C₄,
Y représente un groupe ou un atome sélectionné à partir de :
-O-, -S-, -NH-, 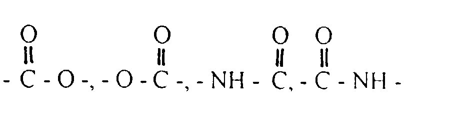
R représente un radical hydrocarbure en C₈ ou supérieur, à chaîne droite ou ramifiée, contenant de façon optionnelle un radical aromatique ou cycloaliphatique substitué ou non substitué,
m est un nombre entier allant de 1 à 4,
p est 0 ou un nombre entier allant de 1 à 10,
en tant que tensioactif ou qu'agent moussant dans une composition tensioactive et/ou moussante.

2. Utilisation d'un composé selon la revendication 1, dans lequel S représente une unité monosaccharide de formule : 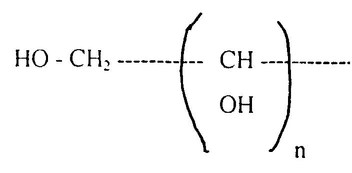 dans laquelle n est un nombre entier allant de 1 à 6

3. Utilisation d'un composé selon la revendication 2, dans lequel n est 4.

4. Utilisation d'un composé selon la revendication 2, dans lequel Y représente O, S ou NH.

5. Utilisation d'un composé selon l'une des revendications précédentes dans lequel X représente un atome d'azote.

6. Utilisation d'un composé selon l'une des revendications précédentes dans lequel m est 3.

7. Utilisation d'un composé selon l'une des revendications précédentes, dans lequel Y représente un atome d'oxygène.

8. Utilisation d'un composé selon l'une des revendications précédentes, dans lequel p est zéro.

9. Utilisation d'un composé selon l'une des revendications précédentes, dans lequel A représente un atome d'hydrogène.

10. Utilisation d'un composé selon l'une des revendications précédentes, dans lequel R représente un groupe alkyle en C₈ à C₂₄.

11. Utilisation d'un composé selon la revendication 10, dans lequel R représente un groupe alkyle en C₁₀ à C₁₄.

12. Utilisation d'un composé selon la revendication 1, qui est un D-gluconamide oxypropylique en C8 à C15.

13. Utilisation d'un composé selon la revendication 1, qui est un D-gluconamide aminopropylique en C₁₆ à C₂₁.

14. Une composition détergente comprenant une quantité détersive et/ou moussante d'un composé de formule I tel que définit dans la revendication 1.

15. Un composé aldonamide monosaccharide de formule la, 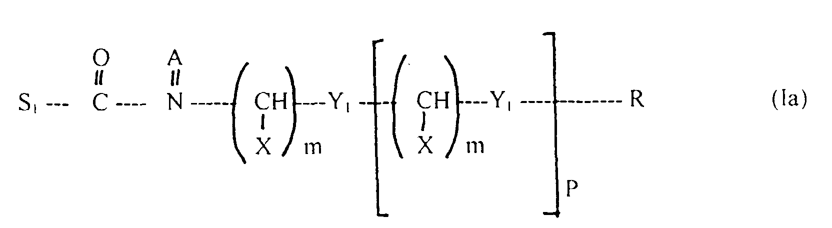 dans laquelle S₁ représente une unité monosaccharide de formule : 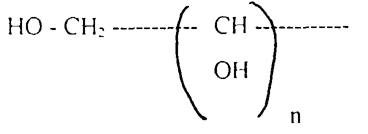 dans laquelle n est un nombre entier allant de 1 à 6
A représente H, un radical hydrocarbure en C₁ - C₈, ou 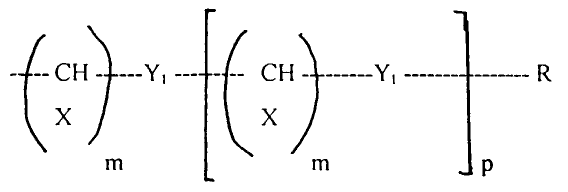
X représente H, ou un groupe alkyle en C₁ - C₄
Y₁ représente - O -, - S - ou - NH -,
R représente un radical hydrocarbure en C₈ ou supérieur, à chaîne droite ou ramifiée, contenant de façon optionnelle un radical aromatique ou cycloaliphatique substitué ou non substitué,
m est un nombre entier allant de I à 4,
p est un nombre entier allant de 1 à 10.
